Europäisches Patentamt

European Patent Office

Office européen des brevets

(19)

(11) Publication number: **0 040 472**
**B1**

(12) **EUROPEAN PATENT SPECIFICATION**

(45) Date of publication of patent specification: **29.01.86**

(51) Int. Cl.⁴: **A 61 K 9/14**

(21) Application number: **81301709.2**

(22) Date of filing: **16.04.81**

(54) **Spray dried N-acetyl-p-aminophenol compositions and method for manufacture thereof.**

(30) Priority: **20.05.80 US 152052**

(43) Date of publication of application:
**25.11.81 Bulletin 81/47**

(45) Publication of the grant of the patent:
**29.01.86 Bulletin 86/05**

(84) Designated Contracting States:
**AT BE CH DE FR GB IT LI LU NL SE**

(56) References cited:
**FR-A-2 150 903**
**GB-A-1 287 431**

**CHEMICAL ABSTRACTS, vol. 92, no. 24, 16th
June 1980, page 328, no. 203522u, Columbus,
Ohio, U.S.A. B. NATH et al.: "Effect of some
formulative and process variables on
dissolution efficiency of paracetamol tablets"**

**Arzneiformenlehre, Dr.rer.nat. Paul Heinz List,
1976, page 74**

**The file contains technical information
submitted after the application was filed and
not included in this specification**

(73) Proprietor: **MALLINCKRODT, INC.**
**675 McDonnell Boulevard P.O. Box 5840**
**St. Louis Missouri 63134 (US)**

(72) Inventor: **Salpekar, Anil M.**
**1421 Jaywood Drive**
**Creve Coeur, MO 63144 (US)**

(74) Representative: **Eyles, Christopher Thomas et al
BATCHELLOR, KIRK & EYLES 2 Pear Tree Court
Farringdon Road
London, EC1R 0DS (GB)**

Courier Press, Leamington Spa, England.

**Description**

The invention relates to spray dried compositions comprising agglomerates of N-acetyl-*p*-aminophenol in a gelatinized starch matrix and to a method for manufacture thereof.

Unlike N-acetyl-*p*-aminophenol, aspirin can be dry mixed with starch and is easily compressible into tablets. Since N-acetyl-*p*-aminophenol and aspirin are sold competitively as analgesics and antipyretics, it would be desirable if N-acetyl-*p*-aminophenol could be provided in a form which is easily compressible into tablets.

In the past, most N-acetyl-*p*-aminophenol tablets have been prepared from a wet granulation including a binder with the N-acetyl-*p*-aminophenol which is dried, mixed with lubricants and disintegrating agents and compressed into tablets. This process involves many processing steps, each of which is subject to operator error.

Less commonly, N-acetyl-*p*-aminophenol tablets have been prepared from spray dried particles of N-acetyl-*p*-aminophenol coated with gelatin; this material is simply mixed with lubricants, disintegrating agents and auxiliary binders and compressed. Compared to the products of the present invention, such spray dried formulations as are commercially available are lacking in bulk density and are less free flowing, while tablets made therefrom are excessively friable. Such commercially available formulations also tend to be dusty and hygroscopic. Gelatin, unlike starch, is an animal product and chemically varies with its origin. This variation has a deleterious effect on the compression characteristics of the spray dried N-acetyl-p-aminophenol product.

Further teachings as to the use of gelatin as binder will be found in GB—A—1287431, which also teaches use of lactose as binder as an alternative to gelatin.

In GB—A—1390032 (FR—A—2150903) there is disclosed a free-flowing granular material suitable for tabletting, comprising:—

a) more than 80% by weight of an N-acyl-aminophenol or ester thereof, such as N-acetyl-*p*-aminophenol, and

b) from 1.5% to 10%, by weight on the weight of component a), of a water soluble polymeric organic binder, such as polyvinylpyrrolidone. Such a material may further contain:—

c) up to 7%, by weight on the weight of component a), of a starch or derivative thereof. It is envisaged that these materials should be prepared by spray drying an aqueous preparation containing the various ingredients. According to page 3, lines 55 to 60:

"The incorporation of component c) into the aqueous fluid made it easily possible to operate the commercial spray-drying equipment so as to consistently obtain a product having a moisture content of from 0.7% to 1.0% by weight."

Example 1 teaches spray drying of an aqueous fluid containing, per 1000 kg of fluid, 500 kg of paracetamol (i.e. N-acetyl-*p*-aminophenol), 10 kg polyvinylpyrrolidone binder, 0.5 kg potassium sorbate as preservative, and 20 kg soluble starch (Laing 1215) under specified conditions to yield a granular free-flowing material having a moisture content of 0.8 to 1.0% by weight. Examples 2 to 4 teach the use of similar spray-drying conditions on similar aqueous fluids in which the soluble starch is replaced by an equivalent amount (20 kg) of a mixture of soluble starch (Laing 1215) and maize starch or an insoluble starch (Laing Driflo). Although page 4, lines 13 to 16, states that "The soluble starch had been pre-treated with dilute acid to remove most of all of its gelatinizing properties to provide water solubility," it would appear that the soluble starch used in these Examples may in fact have been a gelatinized starch. Example 7 substitutes an insoluble starch (Laing Driflo) for the soluble starch of Example 1 but the insoluble starch used, according to page 4, line 13 of GB—A—1390032, was native starch and, accordingly, was not a pregelatinized starch. Moreover, our tests show that under the spray-drying conditions used in Example 7 no gelatinization of the starch occurs. Although the products of Examples 1 to 4 and 7 of GB—1390032 are spray dried materials containing both N-acetyl-*p*-aminophenol and starch, such materials contain in addition polyvinylpyrrolidone as binder.

The present invention accordingly seeks to provide a spray dried N-acetyl-*p*-aminophenol composition which is compressible into tablets and can be used for filling capsules or the like. It also seeks to provide for the manufacture of such compositions.

According to the invention we provide a method for preparing a spray dried N-acetyl-*p*-aminophenol composition, said method comprising:

(a) forming an aqueous slurry of finely divided N-acetyl-*p*-aminophenol and a matrix forming amount of gelatinized starch;

(b) spray drying said slurry to form particles of N-acetyl-*p*-aminophenol in a starch matrix, wherein the starch is the only essential binder present, under spray drying conditions such that the spray dried particles have a moisture content sufficient to prevent the build up of a static charge but less than 1.5 percent by weight.

The invention also provides a method for preparing a spray dried N-acetyl-*p*-aminophenol composition, said method comprising:

(a) forming an aqueous slurry of finely divided N-acetyl-*p*-aminophenol and a matrix forming amount of gelatinized starch;

(b) spray drying said slurry to form particles of N-acetyl-*p*-aminophenol in a starch matrix, wherein the starch is essentially the only binder present, under spray drying conditions such that the spray dried particles have a moisture content sufficient to prevent the build up of a static charge but less than 1.5 percent by weight.

The invention further provides a composition comprising spray dried particles of N-acetyl-*p*-aminophenol in a starch matrix wherein the moisture content is sufficient to prevent the build up of a static charge but less than 1.5 percent by weight, said starch being gelatinized, and wherein the gelatinized starch is the only essential binder present. Yet again, it provides a composition comprising spray dried particles of N-acetyl-*p*-aminophenol in a starch matrix wherein the moisture content is between 0.3 and 1.5 percent by weight, said starch being gelatinized, and wherein the gelatinized starch is essentially the only binder present.

Spray dried compositions of the present invention comprise N-acetyl-*p*-aminophenol in a gelatinized starch matrix; such compositions are formed by spray-drying a slurry containing N-acetyl-*p*-aminophenol and gelatinized starch, said starch being present in a matrix forming amount such that, when the composition is spray dried as described hereinafter, particles of general spherical shape are formed. Generally the gelatinized starch does not comprise more than 25 percent by weight of the aforementioned composition but less than 1.5 percent by weight in most cases will not form an effective matrix. If the spray dried composition is to be tabletted, the gelatinized starch is effectively present in an amount from 7 to 12 percent by weight and more preferably from 7 to 10 percent by weight. If the spray dried composition is to be used for filling capsules or the like, the gelatinized starch may be present in a lesser amount.

The preferred binder is pregelatinized starch USP.

The N-acetyl-*p*-aminophenol, in the form of a finely divided powder (i.e. a powder of particle size sufficiently fine for spray drying), may be added to a solution or dispersion of the starch. For ease of incorporation, however, the starch is pre-blended with an equal amount of N-acetyl-*p*-aminophenol and charged into a suitable high shear mixing device prefilled with water in an amount sufficient to provide a smooth slurry. With continued mixing, the remaining N-acetyl-*p*-aminophenol is added and mixing further continued until a smooth slurry is formed. The minimum amount of water should be used such that the slurry is pumpable. For this purpose, the slurry should have a solids content from 40 to 60 percent by weight and preferably no more than 50 percent. Greater amounts of water can be used of course, but the process becomes less economic since substantially all of the water must be removed during spray drying and if lesser amounts are used such that the solids content is greater than 60 percent by weight, the slurry is not easily handled with commonly available equipment.

The particle size of the N-acetyl-*p*-aminophenol has an effect on the spray dried product. For example, it has been found that if more than 50 percent by weight of the N-acetyl-*p*-aminophenol particles are larger than 75 µm (200 mesh) (U.S. Standard Sieve), then the spherical conformation of the spray dried particles is adversely effected. For use in the present invention, preferably all of the N-acetyl-*p*-aminophenol particles will pass through a 75 µm (200 mesh) screen, more preferably 75 percent will pass through a 45 µm (325 mesh) screen and most preferably all will pass through a 45 µm (325 mesh) screen.

The slurry is then spray dried under spray drying conditions such that particles of N-acetyl-*p*-aminophenol in a starch matrix are formed. The size of the spray dried particles is tailored to the particular application. If the spray dried product is to be compressed into tablets, preferably 80 percent of the particles are coarser than 75 µm (200 mesh) while 95 percent are finer than 425 µm (40 mesh).

The water content of the spray dried particles also has an effect on the product. If the product is to be compressed into tablets, it is preferred that it have a moisture content in the range of 0.3 to 1.5 percent by weight water. If more than 1.5 percent by weight water is left in the product, then it is too wet to be free flowing and may support bacterial growth and if less than 0.3 percent is left, the compressibility of the product is adversely effected and the friability of tablets made from this material increases. If the product is to be used for filling capsules, the spray dried product may contain less than 0.3 percent water but, even then, sufficient water must be left in the product to prevent the build up of a static charge which must be prevented to provide good flow.

Moisture retaining agents can be added to the slurry before it is spray dried to maintain the moisture content. Higher levels of starch will also aid in retaining moisture.

Suitable spray drying conditions depend upon the nature of the spray-drying equipment, whether cocurrent or countercurrent, its size and configuration, the mode of atomization and like factors. Necessary atomization pressure, inlet and outlet temperature and air flow will also depend on the solids content of the slurry and the particle size and moisture content wanted in the spray dried product. Balancing of the several variables can be accomplished by those skilled in the art in view of the following examples. In general, however, it is preferred that the outlet temperature be kept below 150°C to avoid yellowing the product and is preferably maintained as low as possible.

Other active ingredients can be added to the slurry containing N-acetyl-*p*-aminophenol before it is spray dried as long as they are compatible with the starch binder and with N-acetyl-*p*-aminophenol and can withstand spray drying conditions. Suitable materials satisfying these conditions include alkaloids and their salts such as codeine phosphate. Alternatively, other active ingredients may be dry blended with the spray dried particles of N-acetyl-*p*-aminophenol and formed into dosage units. One advantage in adding the other active ingredients to the N-acetyl-*p*-aminophenol before it is spray dried is that the active ingredients are bonded together in the spray dried particles so that the active ingredients do not separate on shipping.

The spray dried particles of the present invention may be combined with still other pharmaceutically acceptable materials and made into the desired dosage form. The product may be used, as is, for filling capsules but if it is to be compressed into tablets, it is preferred that lubricants and disintegrants be added before the material is tabletted. Typical lubricants include polyethylene glycols, stearic acid, magnesium stearate, calcium stearate, zinc stearate, hydrogenated vegetable oils or talc. Suitable disintegrants include alginic acid, sodium alginate, starch and sodium carboxymethyl starch.

The following examples illustrate the invention.

Example 1

Pregelatinized starch in the amount indicated in Table I was mixed with an equal quantiy of N-acetyl-p-aminophenol powder and charged to a high shear mixer containing the indicated amount of water. The N-acetyl-p-aminophenol powder had a particle size such that all of it passed through a 75 μm (200 mesh) screen and 75 percent passed through a 45 μm (325 mesh) screen. Additional N-acetyl-p-aminophenol powder was added such that the resultant mixture contained the indicated amount of N-acetyl-p-aminophenol and mixing continued until a smooth slurry was formed.

TABLE I

| Ingredients | Composition Parts by weight | | | |
|---|---|---|---|---|
| Pregelatinized Starch, USP | 1.5 | 5 | 7 | 10 |
| N-acetyl-p-aminophenol | 98.5 | 95 | 93 | 90 |
| H$_2$O | 100 | 122 | 122 | 122 |

The resultant slurry was spray dried under the conditions shown in Table II.

TABLE II
Spray dryer type

Countercurrent:

Disc Atomizer
    Inlet Temperature                           220°C.

    Outlet Temperature                       90°C.

    Atomization Pressure                41.4 bar (600 psi)

Nozzle Atomizer
    Inlet Temperature                           220°C.

    Outlet Temperature                       85°C.

    Atomization Pressure                41.4 bar (600 psi)

Cocurrent:

Nozzle Atomizer
    Inlet Temperature                           260°C (500°F)

    Outlet Temperature                       66°C (150°F)

    Atomization Pressure                100 bar (1450 psi)

Satisfactory countercurrent conditions were found for disc atomizers with an atomization pressure between 41.4—62.1 bar (600—900 psi), an inlet temperature between 170—260°C. and an outlet temperature between 60—120°C. Corresponding conditions for nozzle atomizers were between 180—260°C. for the inlet temperature and 70—120°C. for the outlet temperature. Suitable cocurrent conditions were found for nozzle atomizers with an atomization pressure between 89.6—117.2 bar (1300—1700 psi), an inlet temperature between 238°—299°C (460—570°F) and an outlet temperature between 60°—82°C (140—180°F).

Spray dried product was evaluated for particle size, flow properties and bulk density as shown in Table III.

TABLE III

| Particle size* % cumulative | Percent by weight Pregelatinized starch/N-acetyl-*p*-aminophenol | | | |
|---|---|---|---|---|
| | 1.5/98.5 | 5/95 | 7/93 | 10/90 |
| >250 μm (+60 m) | 4.9 | 20.5 | — | — |
| >180 μm (+80 m) | 39.6 | 63.1 | 15.5 | 56.5 |
| >150 μm (+100 m) | 56.2 | 80.9 | 36.2 | 73.5 |
| >160 μm (+140 m) | — | 95.6 | 71.1 | 94.6 |
| >75 μm (+200 m) | 94.7 | 98.9 | 86.6 | 99.6 |
| >45 μm (+325 m) | — | — | — | — |
| >38 μm (+400 m) | — | — | — | — |
| <38 μm (−400 m) | 100.0 | 100.0 | 100.0 | 100.0 |
| Flow Properties gm/s | 17.3** | good | good | good |
| Bulk Density gm/cm³ | 0.346 | 0.382 | 0.415 | 0.417 |

\* Particle size was measured with a Sonic Sifter, Model L3P sold by ATM Corporation of Milwaukee, Wisconsin.

\*\* Good.

The spray dried material was tabletted and compared with material prepared by conventional procedures as shown in Table IV.

TABLE IV

| Percent by weight Pregelatinized starch/ N-acetyl-*p*-aminophenol | Compressive force (kg) | Max hardness (kg) | Friability (%) |
|---|---|---|---|
| Spray Drying 5/95 | 560 | 5.6 | — |
| 7/93 | 950 | 10.4 | 0.28 |
| 10/90 | 1150 | 14.8 | 0.2 |
| Glatt Fluid Bed Granulation 5/95 | 1070 | 1.7 | — |
| 10/90 | 1200 | 8.1 | — |
| Conventional Wet Granulation 10/90 | 950 | 5.1 | 1.0 |

Example 2

Spray dried product as described in Example 1 comprising 7% by weight pregelatinized starch and 93% by weight N-acetyl-*p*-aminophenol was dried to the moisture content indicated in Table V and compressed into tablets. The effect of moisture on tablet hardness and friability is also indicated.

5

TABLE V

| Sample No. | Moisture content (%) | Compressive force (kg) | Tablet hardness (kg) | Friability (%) |
|---|---|---|---|---|
| Control | 0.72 | 670 | 8.1 | 0.96 |
| | 0.72 | 970 | 11.3 | 0.27 |
| 1 | 0.60 | 555 | 6.0 | 1.55 |
| | 0.60 | 960 | 9.8 | 2.10 |
| 2 | 0.54 | 560 | 5.1 | 1.71 |
| | 0.54 | 930 | 8.8 | 0.60 |
| 3 | 0.43 | 565 | 4.7 | 1.97 |
| | 0.43 | 750 | 6.5 | 1.03 |
| 4 | 0.26 | 560 | 4.6 | 2.70 |
| | 0.26 | 935 | 7.5 | 3.80 |

Example 3

Spray dried product as described in Example 1 comprising 10% by weight pregelatinized starch and 90% by weight N-acetyl-*p*-aminophenol was dried to the moisture content indicated in Table VI and compressed into tablets. The effect of moisture on tablet hardness and friability is also indicated.

TABLE VI

| Sample No. | Moisture content (%) | Compressive force (kg) | Tablet hardness (kg) | Friability (%) |
|---|---|---|---|---|
| Control | 0.75 | 560 | 6.6 | 0.74 |
| | 0.75 | 950 | 10.9 | 0.29 |
| 1 | 0.66 | 570 | 6.3 | 1.41 |
| | 0.66 | 940 | 10.2 | 0.32 |
| 2 | 0.57 | 565 | 5.7 | 1.60 |
| | 0.57 | 935 | 9.5 | 0.48 |
| 3 | 0.48 | 565 | 4.7 | 1.28 |
| | 0.48 | 945 | 7.9 | 0.66 |
| 4 | 0.29 | 560 | 4.8 | 2.40 |
| | 0.29 | 935 | 8.3 | 0.80 |

Example 4

Following the procedure of Example 1, when a codeine salt is added to the N-acetyl-*p*-aminophenol and the slurry is spray dried and the dried product then mixed with suitable tabletting excipients, tablets having the following composition ranges can be prepared: N-acetyl-*p*-aminophenol 300—650 mg, codeine salt 15—60 mg and pregelatinized starch 2—25 percent by weight.

**Claims**

1. A method for preparing a spray dried N-acetyl-*p*-aminophenol composition, said method comprising:
(a) forming an aqueous slurry of finely divided N-acetyl-*p*-aminophenol and a matrix forming amount of gelatinized starch;

6

(b) spray drying said slurry to form particles of N-acetyl-*p*-aminophenol in a starch matrix, wherein the starch is the only essential binder present, under spray drying conditions such that the spray dried particles have a moisture content sufficient to prevent the build up of a static charge but less than 1.5 percent by weight.

2. A method for preparing a spray dried N-acetyl-*p*-aminophenol composition, said method comprising:

(a) forming an aqueous slurry of finely divided N-acetyl-*p*-aminophenol and a matrix forming amount of gelatinized starch;

(b) spray drying said slurry to form particles of N-acetyl-*p*-aminophenol in a starch matrix, wherein the starch is essentially the only binder present, under spray drying conditions such that the spray dried particles have a moisture content sufficient to prevent the build up of a static charge but less than 1.5 percent by weight.

3. The method of claim 1 or claim 2, wherein the spray drying conditions are selected such that the spray dried particles have a moisture content in the range of 0.3 to 1.5 percent by weight.

4. The method of any one of claims 1 to 3, wherein the gelatinized starch comprises 7 to 12 percent by weight of said composition.

5. The method of any one of claims 1 to 4, wherein the slurry contains no more than 60 percent by weight solids and wherein said solids comprise 98.5 to 75 percent by weight of N-acetyl-*p*-aminophenol and 1.5 to 25 percent by weight of starch.

6. The method of any one of claims 1 to 5, wherein another compatible active ingredient is substituted for part of the N-acetyl-*p*-aminophenol.

7. The method of claim 6, wherein the active ingredient is an alkaloid or salt thereof.

8. The method of claim 7, wherein the alkaloid salt is codeine phosphate.

9. The method of any one of claims 1 to 8, wherein 100 percent of the finely divided N-acetyl-*p*-aminophenol will pass through a 75 µm (200 mesh) screen and 75 percent will pass through a 45 µm (325 mesh) screen.

10. The method of any one of claims 1 to 9, wherein the gelatinized starch is pregelatinized starch USP.

11. The method of any one of claims 1 to 10, wherein the spray drying conditions are selected so that at least 80 percent of the spray dried particles are coarser than 75 µm (200 mesh) while 95 percent are finer than 425 µm (40 mesh).

12. A composition comprising spray dried particles of N-acetyl-*p*-aminophenol in a starch matrix wherein the moisture content is sufficient to prevent the build up of a static charge but less than 1.5 percent by weight, said starch being gelatinized, and wherein the gelatinized starch is the only essential binder present.

13. A composition comprising spray dried particles of N-acetyl-*p*-aminophenol in a starch matrix wherein the moisture content is sufficient to prevent the build up of a static charge but less than 1.5 percent by weight, said starch being gelatinized, and wherein the gelatinized starch is essentially the only binder present.

14. The composition of claim 12 or claim 13, wherein the moisture content is in the range of 0.3 to 1.5 percent by weight.

15. The composition of any one of claims 12 to 14, or claim 12 wherein the gelatinized starch comprises between 1.5 and 25 percent by weight of the solids content.

16. The composition of any one of claims 12 to 15, wherein the gelatinized starch comprises between 7 and 12 percent by weight of the solids content.

17. The composition of any one of claims 12 to 16, wherein another compatible active ingredient is substituted for part of the N-acetyl-*p*-aminophenol.

18. The composition of claim 17, wherein the active ingredient is an alkaloid or salt thereof.

19. The composition of claim 18, wherein the alkaloid salt is codeine phosphate.

**Patentansprüche**

1. Verfahren zur Herstellung einer sprühgetrockneten N-Acetyl-*p*-aminophenol-Zusammensetzung, bei dem man

(a) eine wässrige Aufschlämmung von fein zerteiltem N-Acetyl-*p*-aminophenol und einer eine Matrix bildenden Menge gelatinierter Stärke bildet, und

(b) die Aufschlämmung unter Bildung von Teilchen aus N-Acetyl-*p*-aminophenol in einer Stärkematrix mit Stärke als dem einzigen wesentlichen anwesenden Bindemittel unter solchen Sprühtrocknungsbedingungen sprühtrocknet, daß die sprühgetrockneten Teilchen einen zur Verhinderung des Aufbaus einer elektrostatischen Ladung ausreichenden, jedoch weniger als 1,5 Gew.-% betragenden Feuchtigkeitsgehalt haben.

2. Verfahren zur Herstellung einer sprühgetrockneten N-Acetyl-*p*-aminophenol-Zusammensetzung, bei dem man

(a) eine wässrige Aufschlämmung von fein zerteiltem N-Acetyl-*p*-aminophenol und einer eine Matrix bildenden Menge gelatinierter Stärke bildet, und

(b) die Aufschlämmung unter Bildung von Teilchen aus N-Acetyl-*p*-aminophenol in einer Stärkematrix

0 040 472

mit Stärke als dem im wesentlichen einzigen anwesenden Bindemittel unter solchen Sprühtrocknungsbedingungen sprühtrocknet, daß die sprühgetrockneten Teilchen einen zur Verhinderung des Aufbaus einer elektrostatischen Ladung ausreichenden, jedoch weniger als 1,5 Gew.-% betragenden Feuchtigkeitsgehalt haben.

3. Verfahren nach Anspruch 1 oder Anspruch 2, bei dem die Sprühtrocknungsbedingungen so ausgewählt werden, daß die sprühgetrockneten Teilchen einen Feuchtigkeitsgehalt in dem Bereich von 0,3 bis 1,5 Gew.-% haben.

4. Verfahren nach einem der Ansprüche 1 bis 3, bei dem die gelatinierte Stärke 7 bis 12 Gew.-% der genannten Zusammensetzung beträgt.

5. Verfahren nach einem der Ansprüche 1 bis 4, bei dem die Aufschlämmung nicht mehr als 60 Gew.-% Feststoffe enthält und die genannten Feststoffe 98,5 bis 75 Gew.-% N-Acetyl-*p*-aminophenol und 1,5 bis 25 Gew.-% Stärke aufweisen.

6. Verfahren nach einem der Ansprüche 1 bis 5, bei dem man einen Teil des N-Acetyl-*p*-aminophenols gegen einen anderen verträglichen aktiven Bestandteil austauscht.

7. Verfahren nach Anspruch 6, bei dem der aktive Bestandteil ein Alkaloid oder dessen Salz ist.

8. Verfahren nach Anspruch 7, bei dem das Alkaloidsalze Kodeinphosphat ist.

9. Verfahren nach einem der Ansprüche 1 bis 8, bei dem 100% des fein zerteilten N-Acetyl-*p*-aminophenols durch ein 75 µm Sieb (200 Mesh) und 75% durch ein 45 µm Sieb (325 Mesh) hindurchgehen.

10. Verfahren nach einem der Ansprüche 1 bis 9, bei dem die gelatinierte Stärke vorgelatinierte Stärke USP ist.

11. Verfahren nach einem der Ansprüche 1 bis 10, bei dem man die Sprühtrocknungsbedingungen so auswählt, daß wenigstens 80% der sprühgetrockneten Teilchen größer als 75 µm (200 Mesh) sind, während 95% feiner als 425 µm (40 Mesh) sind.

12. Zusammensetzung mit sprühgetrockneten Teilchen von N-Acetyl-*p*-aminophenol in einer Stärkematrix, in welcher der Feuchtigkeitsgehalt zur Verhinderung des Aufbaus einer elektrostatischen Ladung ausreicht, aber kleiner als 1,5 Gew.-% ist, wobei die Stärke gelatiniert ist und die gelatinierte Stärke das alleinige, wesentliche, anwesende Bindemittel ist.

13. Zusammensetzung aus sprühgetrockneten Teilchen von N-Acetyl-*p*-aminophenol in einer Stärkematrix, in welcher der Feuchtigkeitsgehalt zur Verhinderung des Aufbaus einer elektrostatischen Ladung ausreicht, aber kleiner als 1,5 Gew.-% ist, wobei die Stärke gelatiniert ist und die gelatinierte Stärke im wesentlichen das alleinige anwesende Bindemittel ist.

14. Zusammensetzung nach Anspruch 12 oder Anspruch 13, bei der der Feuchtigkeitsgehalt in dem Bereich von 0,3 bis 1,5 Gew.-% liegt.

15. Zusammensetzung nach einem der Ansprüche 12 bis 14 oder Anspruch 12, bei der die gelatinierte Stärke zwischen 1,5 und 25 Gew.-% des Feststoffgehalts beträgt.

16. Zusammensetzung nach einem der Ansprüche 12 bis 15, bei der die gelatinierte Stärke zwischen 7 und 12 Gew.-% des Feststoffgehalts beträgt.

17. Zusammensetzung nach einem der Ansprüche 12 bis 16, bei der ein Teil des N-Acetyl-*p*-aminophenols durch einen anderen verträglichen aktiven Bestandteil ausgetauscht ist.

18. Zusammensetzung nach Anspruch 17, in der der aktive Bestandteil ein Alkaloid oder dessen Salz ist.

19. Zusammensetzung nach Anspruch 18, bei der das Alkaloidsalz Kodeinphosphat ist.

**Revendications**

1. Procédé pour préparer une composition de N-acétyl-*p*-aminophénol séchée par pulvérisation qui consiste:
(a) à former une bouillie aqueuse comprenant du N-acétyl-*p*-aminophénol finement divisé et une quantité pour former une matrice d'un amidon gélatinisé;
(b) à sécher par pulvérisation cette bouillie de manière à former des particules de N-acétyl-*p*-aminophénol dans une matrice d'amidon, où l'amidon est le seul liant essentiel présent, dans des conditions de séchage par pulvérisation telles que les particules séchées par pulvérisation ont une teneur en humidité suffisante pour prévenir l'accumulation d'une charge statique, mais cependant inférieure à 1,5% en poids.

2. Procédé pour préparer une composition de N-acétyl-*p*-aminophénol séchée par pulvérisation qui consiste:
(a) à former une bouillie aqueuse de N-acétyl-*p*-aminophénol finement divisée et d'une quantité d'amidon gélatinisé adaptée pour former une matrice;
(b) à sécher cette bouillie par pulvérisation, afin de former des particules de N-acétyl-*p*-aminophénol incorporées dans une matrice d'amidon, l'amidon étant essentiellement le seul liant présent, dans des conditions de séchage par pulvérisation telles que les particules séchées par pulvérisation ont une teneur en humidité suffisante pour prévenir l'accumulation d'une charge statique, mais cependant inférieure à 1,5% en poids.

3. Procédé selon la revendication 1 ou 2, caractérisé en ce qu'on sélectionne les conditions du séchage par pulvérisation, de façon que les particules séchées aient une teneur en humidité comprise entre 0,3 et 1,5% en poids.

8

4. Procédé selon l'une quelconque des revendications 1 à 3, caractérisé en ce que l'amidon gélatinisé comprend entre 7 et 12% en poids de ladite composition.

5. Procédé selon l'une quelconque des revendications 1 à 4, caractérisé en ce que la bouillie ne contient pas plus de 60% en poids de matières solides et en ce que lesdits solides comprennent entre 98,5 et 75% en poids de N-acétyl-*p*-aminophénol et de 1,5 à 25% en poids d'amidon.

6. Procédé selon l'une quelconque des revendications 1 à 5, caractérisé en ce qu'on substitue à une partie du N-acétyl-*p*-aminophénol, une autre substance active compatible.

7. Procédé selon la revendication 6, caractérisé en ce que la substance active est un alcaloïde ou un sel de celui-ci.

8. Procédé selon la revendication 7, caractérisé en ce que le sel d'alcaloïde est le phosphate de codéïne.

9. Procédé selon l'une quelconque des revendications 1 à 8, caractérisé en ce que la totalité du N-acétyl-*p*-aminophénol finement divisé traverse un tamis dont les mailles ont 75 µm (200 mesh), tandis que 75% de celui-ci traverse un tamis de 45 µm (325 mesh).

10. Procédé selon l'une quelconque des revendications 1 à 9, caractérisé en ce que l'amidon gélatinisé est un amidon prégélatinisé USP.

11. Procédé selon l'une quelconque des revendications 1 à 10, caractérisé en ce que l'on sélectionne les conditions du séchage par pulvérisation, de façon qu'au moins 80% des particules séchées par pulvérisation aient une granulométrie supérieure à 75 µm (200 mesh), tandis que 95% d'entre elles sont plus fines que 425 µm (40 mesh).

12. Composition comprenant des particules séchées par pulvérisation de N-acétyl-*p*-aminophénol dans une matrice d'amidon, caractérisée en ce que la teneur d'humidité est suffisante pour prévenir l'accumulation d'une charge statique, tout en étant inférieure à 1,5% en poids, ledit amidon étant gélatinisé, et dans laquelle l'amidon gélatinisé est le seul liant essentiel présent.

13. Composition comprenant des particules séchées par pulvérisation de N-acétyl-*p*-aminophénol dans un matrice d'amidon où la teneur en humidité est suffisante pour prévenir l'accumulation d'une charge statique, tout en étant cependant inférieure à 1,5% en poids, ledit amidon étant gélatinisé, et où cet amidon gélatinisé est essentiellement le seul liant présent.

14. Composition selon la revendication 12 ou 13, caractérisée en ce que le taux d'humidité se situe entre 0,3 et 1,5% en poids.

15. Composition selon l'une quelconque des revendications 12 à 14 ou 13, où l'amidon gélatinisé représente entre 1,5 et 25% en poids de la teneur en matière solides.

16. Composition selon l'une quelconque des revendications 12 à 15, caractérisée en ce que l'amidon gélatinisé représente entre 7 et 12% en poids de la teneur en matières solides.

17. Composition selon l'une quelconque des revendications 12 à 16, caractérisée en ce qu'une autre substance active compatible a été substituée à une partie du N-acétyl-*p*-aminophénol.

18. Composition selon la revendication 17, caractérisée en ce que la substance active est un alcaloïde ou un sel d'alcaloïde.

19. Composition selon la revendication 18, caractérisée en ce que le sel d'alcaloïde est le phosphate de codéïne.